Europäisches Patentamt

European Patent Office

Office européen des brevets

(11) Publication number: **0 499 662 B1**

## EUROPEAN PATENT SPECIFICATION

(45) Date of publication of patent specification: **30.11.94**

(51) Int. Cl.5: **A61L 15/22**, A61M 35/00, A61K 47/14

(21) Application number: **91102354.7**

(22) Date of filing: **19.02.91**

(54) **Compositions with enhanced penetration.**

(43) Date of publication of application:
**26.08.92 Bulletin 92/35**

(45) Publication of the grant of the patent:
**30.11.94 Bulletin 94/48**

(84) Designated Contracting States:
**AT BE CH DE DK ES FR GB GR IT LI LU NL SE**

(56) References cited:
EP-A- 0 174 108      EP-A- 0 196 769
EP-A- 0 272 918      EP-A- 0 332 147
WO-A-88/02256       WO-A-90/06736

(73) Proprietor: **WARNER-LAMBERT COMPANY**
**201 Tabor Road**
**Morris Plains New Jersey 07950 (US)**

(72) Inventor: **Mahjour, Majid**
**82 Koclas Drive**
**Netcong, New Jersey 07857 (US)**
Inventor: **Mauser, Bernadette**
**462 Kingsland Avenue**
**Lyndhurst, New Jersey 07071 (US)**
Inventor: **Linn, Edwards Eonchao**
**15 Sehirra Drive**
**Wanaque, New Jersey 07465 (US)**
Inventor: **Rashidbaigi, Zahra**
**33 Ferncliff Road**
**Morris Plains, New Jersey 07950 (US)**

(74) Representative: **Mansmann, Ivo**
**Gödecke AG**
**Patentwesen**
**Mooswaldallee 1-9**
**D-79090 Freiburg (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The instant invention covers a carrier of drugs, a new delivery device, which is a dual-ply laminate of a silicone elastomer and a macroporous polyethylene slab. It is especially useful for transdermal delivery of drugs that require a large maintenance dose.

EP-A-0 332 147 describes transdermal compositions with a penetration-enhancing system for an improved penetration of various drugs through living membranes such as the human skin. This transmembranally administrable composition comprises

a.) about 0.2% to about 30% of a therapeutically active medicament or bioaffecting agent,

b.) up to about 99.8% of a solvent and, if desired

c.) up to about 15% of a gelling agent.

The use of solvents and gelling agents as a carrier for the active medicament or bioaffecting agent has certain disadvantages especially if higher doses are required. The objective of the present invention is to develop a more effective delivery system which is especially useful for transdermal delivery of medicaments that require a large maintenance dose and an especially uniform delivery over long periods of time. A most exact delivery control is desired in order to avoid undesirable side effects due to such factors as the failure of the drug to sufficiently or uniformly penetrate the cutaneous membrane and enter the body in the desired amount to produce therapeutic systemic effects. There is also the risk of dose dumping and other undesired side effects. It has already been suggested to use certain membranes as controlling means for transmembranally active pharmaceutical compositions.

US-A- 4 331 651 covers in part, caprylic/capric acid -1,2-propanediol-diester used as a release-promoting substance used in a silicon rubber carrier for an active ingredient. US-A- 4 336 234 covers a microsealed transdermal delivery pad for nitroglycerin administration which contains a silicon matrix having microsealed compartments of silicone rubber mixed with a hydrophilic solvent system, the solvent system can contain a saturated coconut oil such as Miglyol which improves the transport and absorption of the nitroglycerin.

US-A- 4 788 063 covers a transdermal pharmaceutical composition for delivery of cholinergic and anticholinergic basic drugs. The active ingredients are in a vehicle such as lower fatty acid which serves as a solvent and a transdermal delivery agent.

US-A- 4 485 087 covers a process for preparing a composite pharmaceutical preparation.

EP-A- 0 243 891 covers a laminar structure for administering a chemical at a controlled rate.

In EP-A- 0 332 147 certain controlling membranes are contemplated. The use of membranes, especially microporous membranes, such as silicone membranes or polyurethane membranes at the filing date of the above document constituted certainly a technical advantage. The delivery control however of the compositions described in the above mentioned documents is still not yet perfect.

It has now been found that the laminated combinatin of two different layers, one of them having a macroporous structure, exerts an unexpectedly improved and unexpectedly uniform percutaneous release pattern of various medicaments.

The two-ply composite laminate provides an ideal carrier for transdermal delivery of a drug, especially in situations that require large amounts of medicaments, such as 10 mg per application per day. The gradual release of a medicament via the laminated membranal tissue minimizes the risk of dose dumping and other side effects.

In addition, the use of the present invention results in a reduction in overall drug load dose. Furthermore the laminated transmembranal devices of the present invention generally serve as an aid to the patient to administer the proper dose.

Moreover, the present invention provides a permeation enhancer which is nonirritating to skin.

Generally the two-ply composite laminate comprises a layer I which contains a solution of an active medicament in a solvent which is dispersed in a partially cured elastomer (composition A) and another layer II which is laminated onto layer I and contains an active medicament dissolved in a solvent, the solution being incorporated in a macroporous slab (composition B) with a pore size of 10 to 100 $\mu$m.

Accordingly the invention comprises a two-ply composite laminate for transmembranal administration consisting of

1.) a layer I comprising a component A consisting of a solvent and an active medicament incorporated in a partly cured elastomer,

2.) a layer II laminated onto layer I comprising a component B consisting of a solvent and an active medicament incorporated in a macroporous slab with a pore size of 10 to 100 $\mu$m.

The term solvent is intended to include that portion of the formulation which provides the flux of the active ingredient. The permeating-enhancing solvent functions to assist in the migration of the drug components through the membranes and into the bloodstream. Thus, any agents which function to hasten

2

and/or regulatethe transmembranal passage or systemic release of drugs can be used.

The solvent component will comprise about 5 to about 60% weight-by-weight, preferably about 15 to about 40% weight-by-weight and most preferably from about 20 to about 35% weight-by-weight of the total composition.

The solvent for both compositions A and B may be alcohols and higher alcohol derivatives such as for example ethanol, glycerol, ethylene glycol, propylene glycol, other mono- or polyhydric alcohols, isopropylmyristate and propylene glycol dicaprylate/dicaprate (Miglyol 840) and mixtures thereof.

The Trade Mark Miglyol 840 concerns a propylene glycol-diester of caprylic- and capric acid from coconut oil with the CTFA-name propylene glycol dicaprylate/dicaprate. The combination of Miglyol 840 with ethanol is described in detail in EP-A-0 332 147 and provides an excellent permeation-enhancing system also for the laminate system of the present invention. A preferred mixture is a Miglyol 840:ethanol composition 90:10 to 10:90, preferably 70:30 to 90:10 (weight-by-weight). Especially preferred is a 80:20 preparation.

Conventional adjuvants such as for example colorants, perfumes, stabilizers, and the like can also be employed in the compositions of the present invention.

The elastomer of layer I is preferably consisting of partially cured forms of silicone.

Other macroporous slaps may be used instead Interflo[R], a macroporous polyethylene slab. Polyalkylene slabs such as polyethylene and polypropylene are applicable. But also aromatic polymers and polymer mixtures such as polystyrene may be used. Preferred are polyalkylene slabs. Especially preferred as an slab is polyethylene.

The pore size should be in the range of 10 to 100 $\mu$m, preferably 30 to 40 $\mu$m. In use layer I is in contact with the body membrane e.g. the human skin.

The layers I and II should be each about 1.6 mm ( 1/16 inch) thick. Acceptable ranges are 1 to 2 mm. This means that the laminated form is 2 to 4 mm, preferably about 3.2 mm thick.

A preferred laminate is composed of a silicone elastomer forming layer I and a macroporous polypropylene slab forming layer II.

The active ingredient may be any desired drug such as 1,2,3,4,-tetrahydro-9-acridinamine (THA, tacrine), hydrocortisone, diltiazem, tazifyllin, diphenhydramine, hydrocortisone, verapamil, 2-methoxy-[2-(5-methyl 1H-pyrazol-3-yl)ethyl]phenol, an estrogen or a progestin, preferably tacrine. While the above drugs are orally highly efficacious, their use is subject to such problems as dose dumping and high drug use requirements.

Tacrine is a very old compound known to have anticholinesterase activity. It has been shown that this medicament improves the amnesia characteristic of Alzheimer's disease (Brinkman and Gershon, 1983; Flood et al, 1985; Rathman and conner, 1984; MvGeer, 1984).

A cognition activator, as for example tacrine or other amine-like medicament could interfere with the curing process of the silastic fluids to form a silicone adhesive matrix for delivery of drugs across a membrane such as skin. A preferred delivery device of the present invention contains tacrine.

Preferred ranges of tacrine in the carrier are:

Active ingredient, 10-15% weight-by-weight; propylene glycol 1-5% weight-by-weight and the more preferred is 2-3% weight-by-weight; propyleneglycol dicaprylate/dicaprate 1-99% weight-by-weight and the more preferred is 60-80%; ethyl alcohol (95%) 0-99% and the more preferred is 20-40%. Preferred ranges for any of the amine-like drugs used in the carrier are 1-20% weight-by-weight of the active ingredient, preferably 4-15% weight-by-weight, propylene glycol 1-5% weight-by-weight, preferably 2-3% weight-by-weight; propylene glycol dicaprylate/dicaprate (Miglyol 840) 5-70% weight-by-weight, preferably 60-80% weight-by-weight.

Preferred ranges for the steroids are as usually prescribed by one skilled in the art.

The following Example illustrates a preferred embodiment of the invention.

Example 1:

Preparation of CLM Transdermal Patches

A composite laminate matrix (CLM) transdermal system was prepared as follows:

A silicone elastomer base was first prepared by mixing 50 parts of silicone MDX4-4210 and 5 parts of its curing agent MDX4-4210 and 80 parts of silicone fluid 360. Eight grams of this silicone base were mixed thoroughly with 0.2 g of tacrine base and 1.8 g of propylene glycol in a mixer. The mixture was then cast onto a hot plate of approximately 65 cm$^2$ in surface area and heated at 160°C for 2 to 3 minutes before lamination with the Interflo[R] slab. Since the silicone elastomer was only partially cured, the laminated composite matrix was further heated on the same hot plate for an additional 2 minutes before cooling the system to the ambient temperature in a stream of cold water.

A stock solution containing 2.0 g of tacrine base in 10 ml of a binary solvent system consisting of 20 parts of 95% ethanol and 80 parts of Miglyol[(R)] 840 was prepared with mild heating at 60°C. After all the solids were dissolved, the solution was evenly spread over the Interflo[(R)] surface which was approximately the same size as the silicone elastomer cast. Until all the drug solution was absorbed completely into the polymer slab, the Interflo[(R)] side of the composite laminate was further laminated with an impermeable aluminium backing to assure uni-diretional diffusion of tacrine molecules. The composite laminate was finally die cut into a number of circular discs with an average surface area 0.28 cm$^2$. The total amount of tacrine contained in each one of the discs was found to be about 55 mg of tacrine base by HPLC analysis. The analysis was done by first extracting the disc with a binary solvent system, chloroform : acetonitrile (2 : 1), then evaporating the solvent to dryness before injecting into the HPLC.

Pharmaceutical compositions containing Miglyol neutral oils are useful in effecting transdermal delivery of a therapeutic dose of an active drug to the system of mammals. Table I shows a series of comparative in vitro diffusion studies illustrating the usefulness of Miglyol 840 as effectively enhancing the pentration of different drugs across biological membranes. Hairles mouse skin was used.

The in vitro permeation of compounds: atenolol, diltiazem, hydrocortisone, tazifylline, THA, and verapamil from a binary solvent system composed of ethanol and Miglyol 840, as well as from the individual components across hairless mouse skin was evaluated. As Table I indicates, the solvent mixture significantly improved the average flux values (calculated by averaging the flux values obtained at each sampling time) of all the compounds within a 24-hour time period in comparison to that of ethanol or Miglyol alone. The enhancement effect was most profound on tazifylline and hydrocortisone permeation. Minimal enhancement effect was observed on the permeation of verapamil from the binary solvent system, as compared to that of Miglyol alone; while an enhancement factor of 4.76 was obtained in comparison to ethanol.

EP 0 499 662 B1

TABLE  I

Effect of EtOH, Miglyol® 840, and EtOH:Miglyol Binary
Solvent System on the Permeation of Various
Compounds Across Hairless Mouse Skin

| Formulation[a] | Avg. Flux[b] $\mu g/cm^2/h$ | Enh. F Mix/EtOH | Enh. F Mix/Mig |
|---|---|---|---|
| A. Atenolol | | | |
| EtOH | 50.52 | -- | -- |
| Mig | 53.73 | -- | -- |
| EtOH:Mig 20:80 | 262.51 | 5.20 | 4.89 |
| B. Diltiazem | | | |
| EtOH | 13.39 | -- | -- |
| Mig | 38.93 | -- | -- |
| EtOH:Mig 20:80 | 59.17 | 4.42 | 1.52 |
| EtOH:Mig | | | |
| 0:100 | 38.93 | | |
| 10:90 | 39.50 | | |
| 20:80 | 59.17 | | |
| 30:70 | 120.10 | | |
| C. Hydrocortisone | | | |
| EtOH | 0.64 | -- | -- |
| Mig | 1.49 | -- | -- |
| EtOH:Mig 20:80 | 10.24 | 16.00 | 6.87 |
| D. Tazifylline | | | |
| EtOH | 6.63 | -- | -- |
| Mig | 30.71 | -- | -- |
| EtOH:Mig 20:80 | 250.56 | 37.79 | 8.16 |
| E. THA | | | |
| EtOH | 558.91 | -- | -- |
| Mig | 155.49 | -- | -- |
| EtOH:Mig 20:80 | 1476.67 | 2.64 | 9.50 |
| F. Verapamil | | | |
| EtOH | 4.80 | -- | -- |
| Mig | 22.07 | -- | -- |
| EtOH:Mig 20:80 | 22.85 | 4.76 | 1.04 |

[a] The formulations used in this study were saturated with the corresponding drug except for verapamil.
[b] Calculated by averaging the flux values obtained at each sampling time within a 24-hour time period.

## Claims

1. A two-ply composite laminate for transmembranal administration consisting of

5

A.) a layer I comprising a component A consisting of a solvent and an active medicament incorporated in a partly cured elastomer,

B.) a layer II laminated onto layer I comprising a component B consisting of a solvent and an active medicament incorporated in a macroporous slab with a pore size of 10 to 100 $\mu$m.

2. A composite laminate according to claim 1, wherein component A consists of a partly cured elastomer which contains the active medicament dissolved in propylene glycol and component B consists of a macroporous slab which contains the active medicament dissolved in a mixture of propylene glycol dicaprylate/dicaprate and ethanol.

3. A composite laminate according to claim 1, wherein the partly cured elastomer in component A is silicone and the macroporous slab in component B is consisting of polyethylene.

4. A laminate according to claims 1 to 3, wherein the macroporous slab has a pore size of 10 to 100 $\mu$m preferably 35 $\mu$m.

5. A laminate according to claims 1 to 4, wherein the medicament is 1,2,3,4-tetrahydro-9-acridinamine, hydrocortisone, diltiazem, tazifyllin, diphenhydramine, verapamil, 2-methoxy-[2-(5-methyl 1H-pyrazol-3-yl)ethyl]phenol, an estrogen or a progestin.

6. A laminate according to claims 2 to 5, wherein the propylene glycol dicaprylate/dicaprate : ethanol relationship is 90 : 10 to 70 : 30, preferably 80 : 20 (by weight).

7. A laminate according to claims 1 to 6, wherein the layers I and II are each 1 to 2 mm, preferably 1,6 mm (1/16 inch) thick.

8. A process for preparing a two-ply composite laminate for transmembranal administration consisting of

A.) a layer I comprising a component A consisting of a solvent and an active medicament incorporated in a partly cured elastomer,

B.) a layer II laminated onto layer I comprising a component B consisting of a solvent and an active medicament incorporated in a macroporous slab with a pore size of 10 to 100 $\mu$m,

which comprises

(a) mixing an active medicament with a solvent and a curable elastomer component,

(b) casting the obtained component A - mixture onto a plate to form a partly cured layer I,

(c) dissolving an active medicament in a solvent to obtain a solution,

(d) irrigating the obtained solution into a macroporous slab to obtain component B which forms layer II,

(e) laminating layer II onto layer I to produce the desired laminate and if desired cutting into discs of desired size.

9. A process according to claims 8, wherein the partly cured elastomer in component A is silicon and the macroporous slab in component B is consisting of polyethylene.

10. A process according to claim 8 and 9, wherein component A comprises a mixture of a medicament and a curable silicone fluid with propylene glycol and component B comprises a macroporous polyethylene slab and a medicament dissolved in propylene glycol dicaprylate/dicaprate : ethanol 90 : 10 to 50 : 50, preferably 60 :40 (by weight).

11. A process according to claims 8 to 10, wherein the macroporous slab has a pore size of 10 to 100 $\mu$m, preferably 30 to 40 $\mu$m.

12. A process according to claims 8 to 11, wherein the layers I and II are each 1 to 2 mm, preferably 1,6 mm (1/16 inch) thick.

13. A process according to claims 8 to 12, wherein the medicament is 1,2,3,4-tetrahydro-9-acridinamine, hydrocortisone, diltiazem, tazifyllin, diphenhydramine, hydrocortisone, verapamil, 2-methoxy-[2-(5-methyl 1H-pyrazol-3-yl)ethyl]phenol, an estrogen or a progestin.

6

**14.** A disk consisting of a laminate according to claims 1 to 13 having a surface area of from about 1 to about 50 cm$^2$ containing from about 5 to about 200 mg of a medicament.

**15.** A disc consisting of a laminate according to claim 14 containing from 54 to 56 mg of a medicament within a surface area of 2.8 cm$^2$.

## Patentansprüche

**1.** Ein zweilagiges zusammengesetztes Laminat zur transmembranalen Verabreichung, bestehend aus
A) einer Schicht I, die eine Komponente A umfaßt, die aus einem Lösungsmittel und einem wirksamen Medikament, wobei das Lösungsmittel und das wirksame Medilament in ein teilweise gehärtetes Elastomer inkorporiert ist, besteht;
B) einer Schicht II, die auf der Schicht I auflaminiert ist und eine Komponente B umfaßt, die aus einem Lösungsmittel und einem wirksamen Medikament, wobei das Lösungsmittel und das wirksame Medikament in einen makroporösen Formkörper mit einer Porengröße von 10 bis 100 $\mu$m, inkorporiert sind, besteht.

**2.** Ein zusammengesetztes Laminat nach Anspruch 1, worin die Komponente A aus einem teilweise gehärteten Elastomer, das das wirksame Medikament in Propylenglykol gelöst enthält, und die Komponente B aus einem makroporösen Formkörper, der das wirksame Medikament in einer Mischung von Propylenglykoldicaprylat/dicaprinat und Ethanol gelöst enthält, besteht.

**3.** Ein zusammengesetztes Laminat nach Anspruch 1, worin das teilweise gehärtete Elastomer in Komponente A Silicon ist und der makroporöse Formkörper in Komponente B aus Polyethylen besteht.

**4.** Ein Laminat nach den Ansprüchen 1 bis 3, worin der mikroporöse Formkörper eine Porengrösse von 10 bis 100 $\mu$m, vorzugsweise von 35 $\mu$m, aufweist.

**5.** Ein Laminat nach den Ansprüchen 1 bis 4, worin das Medikament 1,2,3,4-Tetrahydro-9-acridinamin, Hydrocortison, Diltiazem, Tazifyllin, Diphenhydramin, Verapamil, 2-Methoxy-[2-(5-methyl 1H-pyrazol-3-yl)ethyl]phenol, ein Östrogen oder ein Progestin ist.

**6.** Ein Laminat nach den Ansprüchen 2 bis 5, worin das Verhältnis von Propylenglykoldicaprylat/dicaprinat : Ethanol 90 : 10 bis 70 : 30, vorzugsweise 80 : 20, (bezogen auf Gewicht) beträgt.

**7.** Ein Laminat nach den Ansprüchen 1 bis 6, worin die Schichten I und II eine Dicke von jeweils 1 bis 2 mm, vorzugsweise 1,6 mm (1/16 inch) aufweisen.

**8.** Verfahren zur Herstellung eines zweilagigen zusammengesetzten Laminats zur transmembranalen Verabreichung, bestehend aus
A) einer Schicht I, die eine Komponente A umfaßt, die aus einem Lösungsmittel und einem wirksamen Medikament, wobei das Lösungsmittel und das wirksame Medilament in ein teilweise gehärtetes Elastomer inkorporiert ist, besteht;
B) einer Schicht II, die auf der Schicht I auflaminiert ist und eine Komponente B umfaßt, die aus einem Lösungsmittel und einem wirksamen Medikament, wobei das Lösungsmittel und das wirksame Medikament in einen makroporösen Formkörper mit einer Porengröße von 10 bis 100 $\mu$m, inkorporiert sind, besteht,
wobei das Verfahren die folgenden Verfahrensstufen umfasst:
(a) Mischen eines wirksamen Medikamentes mit einem Lösungsmittel und einer härtbaren Elastomerkomponente;
(b) Aufgiessen der erhaltenen Mischung der Komponente A auf eine flachen Unterlage zur Bildung einer teilweise gehärteten Schicht I;
(c) Lösen eines wirksamen Medikamentes in einem Lösungsmittel zur Gewinnung einer Lösung;
(d) Begiessen eines makroporösen Formkörpers mit der erhaltenen Lösung zur Gewinnung der Komponente B, welche die Schicht II bildet;
(e) Auflaminieren der Schicht II auf die Schicht I zur Herstellung des gewünschten zusammengesetzten Laminats; und gewünschtenfalls Schneiden in Scheiben gewünschter Größe.

EP 0 499 662 B1

9. Verfahren nach Anspruch 8, worin das teilweise gehärtete Elastomer in Komponente A Silicon ist und der makroporöse Formkörper in Komponente B aus Polyethylen besteht.

10. Verfahren nach Anspruch 8 und 9, worin die Komponente A eine Mischung eines Medikamentes und eines härtbaren Siliconfluids mit Propylenglykol aufweist und die Komponente B einen makroporösen Polyethylenformkörper und ein Medikament, das in Propylenglykoldicaprylat/dicaprinat : Ethanol im Verhältnis 90 : 10 bis 50 : 50, vorzugsweise 60 : 40 (bezogen auf Gewicht), gelöst ist, aufweist.

11. Verfahren nach den Ansprüchen 8 bis 10, worin der makroporöse Formkörper eine Porengrösse von 10 bis 100 $\mu$m, vorzugsweise 30 bis 40 $\mu$m, aufweist.

12. Verfahren nach den Ansprüchen 8 bis 11, worin die Schichten I und II jeweils eine Schichtdicke von 1 bis 2 mm, vorzugsweise 1,6 mm (1/16 in) aufweisen.

13. Verfahren nach den Ansprüchen 8 bis 12, worin das Medikament 1,2,3,4-Tetrahydro-9-acridinamin, Hydrocortison, Diltiazem, Tazifyllin, Diphenhydramin, Verapamil, 2-Methoxy-[2-(5-methyl 1H-pyrazol-3-yl)ethyl]phenol, ein Östrogen oder ein Progestin ist.

14. Scheibenförmiger Formkörper bestehend aus einem Laminat nach den Ansprüchen 1 bis 13, der eine Oberfläche von etwa 1 bis etwa 50 cm$^2$ aufweist und etwa 5 bis etwa 200 mg eines Medikamentes enthält.

15. Scheibenförmiger Formkörper bestehend aus einem Laminat nach Anspruch 14, die 54 bis 56 mg eines Medikamentes innerhalb eines Oberflächenbereichs von 2,8 cm$^2$ enthält.

**Revendications**

1. Un stratifié composite à deux épaisseurs pour l'administration transmembranaire, consistant en :
   A. une couche I comprenant un composant A consistant en un solvant et un médicament actif incorporés dans un élastomère partiellement durci,
   B. une couche II stratifiée sur la couche I comprenant un composant B consistant en un solvant et un médicament actif incorporés dans une plaque macroporeuse ayant une dimension de pores de 10 à 100 $\mu$m.

2. Un stratifié composite suivant la revendication 1, dans lequel le composant A consiste en un élastomère partiellement durci qui contient le médicament actif dissous dans du propylène glycol et le composant B consiste en une plaque macroporeuse qui contient le médicament actif dissous dans un mélange de dicaprylate/ dicaprate de propylène glycol et d'éthanol.

3. Un stratifié composite suivant la revendication 1, dans lequel l'élastomère partiellement durci dans le composant A est un silicone et la plaque macroporeuse dans le composant B consiste en polyéthylène.

4. Un stratifié suivant les revendications 1 à 3, dans lequel la plaque macroporeuse a une dimension de pores de 10 à 100 $\mu$m, de préférence de 35 $\mu$m.

5. Un stratifié suivant les revendications 1 à 4, dans lequel le médicament est la 1,2,3,4-tétrahydro-9-acridinamine, l'hydrocortisone, le diltiazem, la tazifylline, la diphénhydramine, le verapamil, le 2-méthoxy-[2-(5-méthyl 1H-pyrazol-3-yl)éthyl]-phénol, un oestrogène ou une progestine.

6. Un stratifié suivant les revendications 2 à 5, dans lequel le rapport du dicaprylate/dicaprate de propylène glycol à l'éthanol est de 90/10 à 70/30, de préférence de 80/20 en poids.

7. Un stratifié suivant les revendications 1 à 6, dans lequel les couches I et II ont chacune une épaisseur de 1 à 2 mm, de préférence de 1,6 mm (1/16").

8. Un procédé pour préparer un stratifié composite à deux épaisseurs pour l'administration transmembranaire, consistant en :

A. une couche I comprenant un composant A consistant en un solvant et un médicament actif incorporés dans un élastomère partiellement durci,

B. une couche II stratifiée sur la couche I comprenant un composant B consistant en un solvant et un médicament actif incorporés dans une plaque macroporeuse ayant une dimension de pores de 10 à 100 $\mu$m,

qui comprend :

(a) le mélange d'un médicament actif avec un solvant et un composant élastomère durcissable,

(b) la coulée du mélange du composant A obtenu sur une plaque pour former une couche I partiellement durcie,

(c) la dissolution d'un médicament actif dans un solvant pour obtenir une solution,

(d) l'imprégnation de la solution obtenue dans une plaque macroporeuse pour obtenir le composant B qui forme la couche II,

(e) la stratification de la couche II sur la couche I pour produire le stratifié recherché, et si cela est souhaité, la découpe en disques de dimension désirée.

9. Un procédé suivant la revendication 8, dans lequel l'élastomère partiellement durci dans le composant A est un silicone et la plaque macroporeuse dans le composant B consiste en polyéthylène.

10. Un procédé suivant les revendications 8 et 9, dans lequel le composant A comprend un mélange d'un médicament et d'un fluide silicone durcissable avec du propylène glycol et le composant B comprend une plaque macroporeuse de polyéthylène et un médicament dissous dans un mélange de dicaprylate/dicaprate de propylène glycol et d'éthanol selon un rapport de 90/10 à 50/50, de préférence de 60/40 en poids.

11. Un procédé suivant les revendications 8 à 10, dans lequel la plaque macroporeuse a une dimension des pores de 10 à 100 $\mu$m, de préférence de 30 à 40 $\mu$m.

12. Un procédé suivant les revendications 8 à 11, dans lequel les couches I et II ont chacune une épaisseur de 1 à 2 mm, de préférence de 1,6 mm (1/16").

13. Un procédé suivant les revendications 8 à 12, dans lequel le médicament est la 1,2,3,4-tétrahydro-9-acridinamine, l'hydrocortisone, le diltiazem, la tazifylline, la diphénhydramine, le verapamil, le 2-méthoxy-[2-(5-méthyl 1H-pyrazol-3-yl)éthyl]phénol, un oestrogène ou une progestine.

14. Un disque consistant en un stratifié suivant les revendications 1 à 13, ayant une aire superficielle comprise entre environ 1 et environ 50 cm$^2$ contenant d'environ 5 à environ 200 mg d'un médicament.

15. Un disque consistant en un stratifié suivant la revendication 14, contenant de 54 à 56 mg d'un médicament dans une aire superficielle de 2,8 cm$^2$.